(19) **Europäisches Patentamt / European Patent Office / Office européen des brevets**

(11) **EP 3 381 493 B1**

(12) **EUROPEAN PATENT SPECIFICATION**

(45) Date of publication and mention
of the grant of the patent:
**07.06.2023   Bulletin 2023/23**

(21) Application number: **17163731.7**

(22) Date of filing: **30.03.2017**

(51) International Patent Classification (IPC):
***A61M 5/315*** *(2006.01)*

(52) Cooperative Patent Classification (CPC):
**A61M 5/31568;** A61M 2205/3306;
A61M 2205/3368; A61M 2205/3375;
A61M 2205/3561; A61M 2205/52; A61M 2205/6072;
A61M 2205/8293

(54) **ELECTRONIC UNIT FOR INJECTION DEVICES**

ELEKTRONISCHE EINHEIT FÜR INJEKTIONSVORRICHTUNGEN

UNITÉ ÉLECTRONIQUE POUR DISPOSITIFS D'INJECTION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**03.10.2018   Bulletin 2018/40**

(73) Proprietor: **Ypsomed AG**
**3401 Burgdorf (CH)**

(72) Inventors:
• **SCHNEIDER, Andreas**
**3027 Bern (CH)**
• **URBANEK, Leos**
**3012 Bern (CH)**

(74) Representative: **Meier Obertüfer, Jürg et al**
**Ypsomed AG**
**Brunnmattstrasse 6**
**3401 Burgdorf (CH)**

(56) References cited:
WO-A1-2016/118736      WO-A2-2014/064691
WO-A2-2015/006701

• H-A LOELIGER ET AL: "The Factor Graph
Approach to Model-Based Signal Processing",
PROCEEDINGS OF THE IEEE, IEEE. NEW YORK,
US, vol. 95, no. 6, 1 June 2007 (2007-06-01), pages
1295-1322, XP011189327, ISSN: 0018-9219, DOI:
10.1109/JPROC.2007.896497

EP 3 381 493 B1

**Description**

FIELD OF THE INVENTION

The present invention relates to injection devices or medicament delivery devices for injecting, delivering, administering, infusing or dispensing substances and/or liquids such as insulin or hormone preparations. It departs from a medical injection monitoring and patient support system with an injection device and an electronic unit incorporated in, attachable to, or proximal to the injection device.

BACKGROUND OF THE INVENTION

A variety of diseases exist that require regular treatment by subcutaneous injection of a medicament. By way of example, type-1 and type-2 diabetes can be treated by injection, or administration, of insulin doses, either once or several times per day. Such injections can be performed with the help of injection devices that may be handled by the patients themselves. Typical injection devices include pre-filled multi-dose disposable insulin pens and re-usable insulin pens that allow replacement of an empty medicament container or cartridge by a new one. The insulin dose to be injected may typically be manually selected by turning a dosage knob and observing the actual dialed dose from a dose window or display of the insulin pen. A dose is dispensed by inserting the needle into a suited portion of human skin and by pressing an injection or release button of the insulin pen. In order to monitor the injection of insulin, for instance to prevent false handling of the insulin pen or to keep track of the doses already applied, it is desirable to measure and process information related to a use of the injection device, such as information on the injected insulin type, dose, and circumstances of an injection process.

EP 2781230 discloses an exemplary disposable injection device in the form of an auto-injector for automatically injecting an active agent or a liquid drug. The auto-injector has an elongate casing including a syringe holder part for accommodating an active agent container or pre-filled syringe with an injection needle at a distal end. An injection spring is provided for biasing a piston rod and shifting a piston comprised in the container in order to deliver the active agent. The auto-injector also includes a needle protective sleeve that surrounds the needle in a first position, and that may be axially moved in a proximal direction towards a second position. When the distal end of the auto-injector is pressed onto the skin of a patient the needle protective sleeve is displaced in proximal direction, and a needle sleeve spring is charged or tensioned. A first feedback mechanism includes a start-click element that is accelerated by the relaxing injection spring to abut and generate a start click At the end of a dose delivery an end-click element is released to move in proximal direction under the effect of the needle protective sleeve spring to abut and generate and end-click as a second feedback to the user. Upon removal of the auto-injector from the skin the needle protective sleeve is biased to the first position by the needle sleeve spring, and locked in this position by a locking means generating a lock-click as a third feedback to the user. There likewise is a desire to track a usage of such auto-injectors and to ultimately enable monitoring of patient adherence to a prescribed therapy plan.

WO 2007/107564 proposes to record acoustic signals or vibration signals that may be allocated, in particular, to a dose delivery of the medication or to a dose adjustment process. If a detected "click-sound" falls within a low frequency range, a unit dose of medicament is being set. Similarly, if the detected "click-sounds" fall within a high frequency range a dose of medicament is being expelled from the medication delivery device. By counting the number of clicks during an expel sequence the amount of medicament expelled from the medicament delivery device can easily be calculated.

WO 2013/034716 likewise discloses sensors and a processing unit adapted to identify different sounds generated by different sound generating elements of a drug delivery device. In particular, the spectral range of a first, dose setting click-sound generating element is different from the spectral range of the click-sound generated by a second, dose dispensing sound generating element. Respective dose setting and dose dispensing procedures can be identified by spectral analysis of the sound signals detected by the sensor.

WO 2015/006701 discloses a monitoring system for a drug delivery device such as an inhaler or injector, which includes pre-recorded acoustic signals that correspond to a desired operation of the drug delivery device. The monitoring system compares sound detected by a microphone with a set of specific preloaded acoustic signals and calculates a difference between the detected sound and the set of preloaded acoustic waves. If this difference falls within a pre-specified range, the detected sound is identified as an event. The monitoring system includes a monitor comprising the microphone that may be a separate device attached to the drug delivery device. The monitor may also be a component of a display device interfacing with a user of the drug delivery device, e.g. a smartphone, tablet or laptop computer. The monitoring system can sense duration of drug delivery by listening to the amount of time the delivery takes, e.g. the sound of a motor, the sound of drug flowing through the system, and/or the duration of time from a triggering event to an end event such as a piston hitting a stop.

WO 2015/006701 mentions specific methods of comparing a measured waveform to a reference waveform, including calculating a cross correlation, a residual sum of squares, or another measure of the difference between the sample and reference waveforms. Corresponding calculations can be carried out in either the time domain or the frequency domain, and include event waveform parameters determined during an initial calibration or "training mode". Event waveform parameters may be recalculated based on successfully identified events to keep track of changes over time in the waveform due to wear of the mechanical components of the triggering and actuation mechanisms, changes in the acoustic properties of the device, and/or changes over time in the location of the sensor, in order to evolve the waveform standard accordingly.

WO 2014/064691 discloses comparing a sample train of sound impulses to a reference train of pre-recorded sound impulses to determine an amount of insulin injected. Multiple sound samples for each dose are stored in memory to that purpose. WO 2016/118736 A1 discloses a further prior art monitoring method.

Prior art approaches to injection device monitoring based on identification of acoustic events typically rely on a threshold frequency to distinguish between the spectral ranges of dose-setting click sounds and dose-dispensing click sounds, which may not be sufficient if one is to identify distinct successive dispensing click sounds. In prior art approaches where an absolute difference between a recorded waveform and a reference waveform is calculated and compared to a threshold, this will lead to a process abort once a first reference event has been positively identified. Further reference events are disregarded, and no waveform differences with respect to further candidate events are being evaluated. Again, this may be inadequate in case of successive expel clicks with similar characteristics and no clear frequency discrimination.

## SUMMARY OF THE INVENTION

It is an objective of the invention , which is defined in the independent claims, to enable secure, easy and cost-effective operation of components, devices and systems for the generation, collection and distribution of data associated with the handling or use of injection devices. It is an objective of the invention to monitor, in a simple, reliable manner, a handling or use of an injection device for administering a medicament by way of analyzing acoustic signals from the injection device, specifically in view of discriminating sequentially occurring injection events with comparable acoustic signature. These objectives are achieved by a method of monitoring an injection process, an electronic unit, and a computer program according to the independent claims. Preferred embodiments are evident from the dependent patent claims.

According to the invention, a method of monitoring an injection process that is executed by means of an injection device in the context of a medical injection monitoring and patient support system comprises the following steps in order.

A preparatory step of recording a first and at least a second reference click sound respectively characteristic of, or originating from, a first and a second injection event of the injection process, with the first and second injection event occurring sequentially, one after the other, during regular execution of the injection process. The first and the second event may be one of a start of injection and an end of injection, respectively characterized or accompanied by a click sound of a duration of less than one second.

An initiating step of recording and time-stamping, with a microphone connected to a processing or control unit, and during the injection process to be monitored, a sample sound originating from a device event. This device event will be identified with, or assigned to, the first or the second injection event as per the next steps. The initiating step and the subsequent steps are recurring and typically executed a number of times during an injection process.

An evaluating step of determining, by the processing unit, both a first and a second match measure indicative of a match of the sample sound with the first and the second reference click sound, respectively. The match measure is a log-likelihood ratio resulting from a comparison of the sample sound with a reference sound, based on respective waveforms or approximating functions or parameters thereof, both in an integral manner over the entire length of the data set or point-wise at selected sampling times or data points within the data set, and suitable to indicate a probability that the sample sound and the reference sound originate from the same injection event. The device event is identified as a first injection event if the first match measure exceeds the second match measure, while the device event is identified as a second injection event if the second match measure exceeds the first match measure. Concurrent evaluation of two or more match measures ensures that the best matching injection event is identified even if both match measures exceed a certain threshold.

A concluding step of storing, by the processing unit, the timestamp together with a short event identifier such as a flag or bit of the identified first or second injection event, and optionally together with the corresponding match measure for future reference, and discarding the sample sound data. Retaining exclusively the result of the evaluation step in the form of an event identifier rather than the sound data saves memory space and transmission bandwidth.

The aforementioned preparatory step may be performed by the very same electronic unit as the subsequent, recurring steps, however, it may be preferable to have the preparatory step executed by a microphone and processing unit different from the microphone and processing unit used subsequently, and specifically adapted to the task and/or placed in a laboratory-type environment with limited environmental noise.

In the context of the present invention, any step or activity designated as measuring, recording or collecting of a sound may include one or several of the steps including capturing the quantity with a microphone or transducer to generate an output signal, sampling or digitizing the output signal to generate a discrete-time signal, analog and/or digital integration of a signal, analog and/or digital filtering of a signal by appropriate high-, low-, band-pass or notch filters, compressing, equalizing and/or otherwise processing a signal, and storing a discrete-time signal as an audio frame or file. These steps may be performed by appropriate processing means of the microphone itself, or by a dedicated processing unit wired to the microphone.

In the present context, sound pressure waves as compression waves in air, with frequencies in a frequency range between 20 Hz and 20 kHz audible by human beings, are the primary target. Nevertheless, solid or structure borne waves in a similar frequency range may be captured by appropriate transducers responsive to shear, flexural, and surface oscillations. In principle, any kind of mechanical waves in gases, liquids, and solids including vibration, sound, ultrasound and infrasound, emanating from the operative injection device, may be exploited according to the principles disclosed.

In a preferred embodiment, the method further comprises a refined preparatory step of identifying, from each of the first and second reference sound, a limited number of model, or spectral, parameters corresponding to a chosen model for consolidated representation of the reference sound data. Exemplary model parameters may include dominant frequencies as obtained by Fourier Transform of the respective reference sound waveform, and corresponding amplitudes or coefficients. The number of model parameters, or model order, may be different for the two reference sounds, is generally constrained between 1 and 12, and preferably equals 2. The spectral parameters are subsequently provided to, and stored at, the processing unit for subsequent comparison with the complete, non-parametrized, non-compressed data of a discrete time signal data of the sample sound. Extracting and storing an optimized minimum number of suitably selected spectral parameters for each reference sound ensures that even a larger number of reference injection events, possibly from plural distinct injection devices, may be stored within off-line reach of the processing unit at affordable memory requirements.

In a further preferred embodiment of the invention, the method comprises the step of generating, from the sample sound, a discrete-time signal with a number of data points, each data point comprising a time value and corresponding sample sound data value. This step is followed by a step of determining, for each data point in the discrete-time signal, a local match measure or single point deviation, between the corresponding data value of the discrete-time signal and an evaluation of the first set of model parameters at the respective time value, wherein such evaluation is equivalent to a discrete-time parameter-based reference sound reconstruction. Ultimately the maximum local match measure is declared as the first match measure. This process is subsequently repeated for the second set of model parameters, wherein the maximum local match measure may occur at a different time step.

In a preferred embodiment, the injection device comprises a start-click element generating a Start-of-Injection (SoI) click sound at or near the start of the drug expel process to signal beginning of a piston forward activity as the first injection event, and an end-click element generating an End-of-Injection (EoI) signal at or near the end of the drug expel process in order to signal completion of a drug injection or piston forward activity as the second injection event. The start-click element may be accelerated by a first resilient element to abut and generate a start click, while the end-click element may be released to move under the effect of a second resilient element to abut and generate an end-click as a second feedback to the user. Each resilient element may be a spring, either at least partly pre-charged before the injection process, or at least partly charged by transfer of energy, during the injection process, from the user or from a device-internal source of energy. In this embodiment, the device events pertaining to successively recorded sample sounds are identified as a start and as an end of a drug expel process of the injection process. A time delay between the two injection events is determined by comparing the two respective timestamps, and the drug expel process is concluded to be successful if the time delay is within a certain range and in particular does not exceed a certain threshold.

The invention may be employed for monitoring a use or handling of fix dose injection devices including single-dose injection devices such as auto-injectors or patch injectors as well as multi-dose injection devices such as spring driven autopens or fix dose injectors. Fix-dose injection devices have a single, non-variable dosage volume, or provide a limited number of fixed, non-variable injection dosage volumes for the user to choose from. With fix dose injection devices there is neither a need nor a possibility to detect variable dose settings or injected dose quantities. Dedicated click sounds purportedly designed into the injection device in order to provide an audible feedback signal to the user at a start of an injection or drug delivery, at an end or completion of a drug delivery, or at a lock or unlock sound of a cover sleeve or of a syringe displaceable relative to the device housing, or of a device cap removable from the device housing, are the preferred target sounds in this context. Further acoustic signals indicative of a status change, for instance caused by any kind of moving device components including resilient elements released from a pre-strained state, may likewise be exploited by the invention. Likewise, noise generated by a fluid flowing in a conduit, or by a stopper moving in a cartridge, may be captured and exploited according to the principles disclosed. Ultimately, injection devices with electrical drive or feedback means may generate, via loudspeakers, artificial click or equivalent sounds that in turn may be captured and exploited in accordance with the invention.

In a further preferred embodiment of the invention, the injection device is an auto-injector for automatically delivering a fixed dose of liquid drug from a pre-filled syringe by means of a pre-strained injection spring provided for biasing a piston rod and shifting a piston comprised in the syringe. The auto-injector comprises a needle protective sleeve, or cover sleeve, for protecting a needle of the syringe after removal from the injection site. Upon removal of the auto-injector from the injection site the needle protective sleeve is biased to a needle protecting position by the cover sleeve spring, and locked in this position by a locking means generating a lock-click or locking sound. The locking of the cover sleeve in this final, protective position therefore is a third injection event in turn characterized by a distinct sound providing a third feedback to the user. Hence the detected start and end of a substance delivery may be advantageously combined with an injection device lift-off detection to obtain a characterization of the ongoing injection process. In this embodiment, a third reference signal characteristic of, or originating from, the cover sleeve being locked in a needle protective position is recorded in the preparatory step. A next device event is identified as device removal if the match measure of the next sample sound and the third reference sound exceeds all other match measures. Ultimately, a time delay between the delivery-completed and device-removed injection events is determined by comparing the two respective timestamps, and the injection process is concluded to be successful if the time delay exceeds a minimum holding or dwell time.

In the auto-injector embodiment, each of the resilient elements mentioned previously may be one of the injection spring, the cover sleeve spring, or a further spring either at least partly pre-charged before the injection process, or at least partly charged by transfer of energy, during the injection process, from the injection spring.

The invention may also be employed for monitoring a use or handling of variable dose injection devices that, in addition or alternative to the signaling sounds mentioned above in the context of fix-dose injection devices, generate dose dialing and/or expel clicks. The latter are generally associated with units of medication being set, dialed, corrected, or expelled, and may be caused by rotating ratchet wheels or shifting toothed rods coupled to a dosage knob or drive unit. Again, the acoustic signature of a corresponding single dosage unit click, either for increasing or decreasing a dose being set or corrected, or for expelling a set dose, may be exploited to ultimately identify individual dosage units as injection events, and enable a count there of in order to determine a set or expelled dose. The fact that such dosage unit clicks may be generated in rapid succession, e.g. at a rate of five to twenty clicks per second, and that successive individual dosage unit click sounds may significantly overlap in time, may have to be taken into account.

Accordingly, in an alternative preferred embodiment, for variable dose injection devices the injection events include a dosage unit increase setting, a dosage unit decrease setting, and a dosage unit being expelled or delivered. Reference signals characteristic of, or originating from, at least two of these injection events are recorded in the preparatory step. Each of a series of at least two sample sounds consecutively recorded and time-stamped are identified as originating from a dosage unit expel event. The number of dosage unit expel events is counted and assigned to an effectively delivered dose. Alternatively, consecutive dosage unit increase events and consecutive dosage unit decrease events may be counted individually and subtracted to obtain a corrected set dose.

In this embodiment, monitoring activities include capturing date, time, and dose of an injection process, and wirelessly transmitting that data to a mobile device or end-user gateway. To that purpose, the processing unit accurately determines at least one dosage process and dosage amount from the identified click sounds, wherein appropriate dosage processes include setting of a prescribed dose, correcting a previously set dose, and expelling a set dose. Optionally, the determined dosage amounts are compared and evaluated to identify incomplete or otherwise unsatisfactory dose delivery, or separable priming activities.

In a further advantageous variant of the invention, a wireless communication unit is connected to the processing unit, and adapted to wirelessly communicate, specifically upload, injection information to a nearby mobile device. The injection information includes at least a time stamp and the injection event identifier, indicative of a time and type of an injection event identified by means of its acoustic event signature. For instance, the injection information may indicate that dose delivery start and end events were identified at time t1 and time t2. The injection information may be transmitted instantaneously, or stored in a memory unit connected to the processing unit, for later upload or batch transfer. The injection information may, in addition or alternatively, include consolidated injection information derived from a plurality of identified injection events by the processing unit, such as time, dosage type (set, correct, expel) and dosage amount for a variable-dose injection process. Consolidated injection information may also include a quality measure of an injection process, such as a binary flag indicating successful completion, or failure, of an injection process. Positive quality indication may be concluded based on one or several criteria including a) individual injection events are detected in the expected order, b) fix dose is expelled completely, c) holding or dwell time is sufficient, d) dialed dose corresponds to expelled dose. Restricting transmission to consolidated or quality information saves memory space and bandwidth as compared to an unfiltered transmission of each single identified injection event.

According to the invention, an electronic unit comprising a microphone and a processing unit is adapted to cooperate with an injection device generating a click sound during an injection process, and configured to execute some or all of the aforementioned method steps.

The electronic unit may advantageously be embedded in a mobile device such as a mobile phone, tablet device, laptop computer, or any other portable or wearable smart gadget with Human Machine Interfacing capabilities including a microphone as well as a display and/or loudspeaker to provide feedback or instructions to the user. Prior to an injection process the mobile device is placed sufficiently close to the injection device to enable detection of relevant sounds emanating from the injection device. In this case, no dedicated hardware elements are required, but the electronic unit is rather, and at least conceptually, made up of corresponding constituents of the mobile device, such as a built-in microphone and processing power.

Advantageously, a copy of a computer program stored on a computer-readable medium in the form of a smartphone application program is installed on the mobile device. The computer program causes, when being executed by a processor of the mobile device for monitoring an injection process, the processing unit to execute some or all of the aforementioned method steps. The computer-readable medium may be a floppy disk, a hard disk, an USB (Universal Serial Bus) storage device, a RAM (Random Access Memory), a ROM (Read Only Memory), an EPROM (Erasable Programmable Read Only Memory), or a data communication network, e.g. the Internet, which allows downloading program code.

The electronic unit may advantageously be integrated in the injection device and hence disposed of together with the injection device. For re-usable injection devices with cartridge replacement, this will be the case after expiry of a certain minimum lifetime of the injection device. The inclusion of an electronic unit with a microphone for acoustic tracking in a newly designed injection device may be advantageous over other sensors because of the distance permitted between the signal source and the microphone, allowing the latter to be placed with increased flexibility.

The electronic unit may advantageously be part of a reusable electronic module that attaches to a mechanical injection device in a detachable or removable way, for monitoring of an injection process executed by a user by means of the injection device. This is of particular interest in retrofit configurations with an existing injection device design that is not available for adaptations such as incorporation of an electronic unit. The electronic module including an electronic unit according to the invention is adapted to detect and exploit acoustic signals emanating from the injection device, and may expressly exclude the presence of any further mechanical, optical, and/or electrical sensor for monitoring the use of the injection device. Other kinds of interaction between the electronic module and the injection device may independently be present or be excluded. For instance, a mechanical keying element of the electronic module may interact with dedicated counterpart keying element provided on the injection device to disable activation preventing elements of the injection device, as a prerequisite for a proper use of the injection device.

In a preferred variant of the electronic unit being integrated in the injection device or being part of an electronic module, the electronic unit comprises two microphones at two different locations in the injection device or module. The two locations are axially spaced apart by a distance of at least a diameter of the injection device. Each of the two microphones may thus be placed close to a respective click sound generating element in configurations where the two click sound generating elements are likewise axially offset. Two sample sounds per device event are thus processed according to the aforementioned method, wherein one of the two may be subject to less background noise and hence lead to an unambiguous reference injection event identification. In addition the two sample sounds may be investigated for differences in amplitude and/or peak signal phase, which may provide an additional hint as to the location of the click sound generating element and corresponding injection event.

In a preferred variant of the electronic unit being integrated in the injection device or being part of an electronic module, the electronic unit comprises a visual, audible and/or tactile status indicator indicating to a user a status of the system. The status of the system may include any of a device status of the injection device, a module status of the electronic module, or a process status of an overall injection process or injection device handling process. The status indicator may be simple and limited to a few Light Emitting Diodes LEDs in traffic-light colors and/or an audible signal generator for generating language-independent beep sounds or simple melodies. The status indicator may explicitly exclude any advanced human-machine interfacing capability, and be limited to a few, specifically less than ten, messages conveyable to the user. In particular, the electronic unit may not be wired to, and the electronic module may be devoid of, a display, screen, or projector for visually transmitting readable instructions, and likewise exclude an artificial speech assistant for reading out loud the instructions. Such advanced HMI functionality including elaborate graphic display and speech output capabilities are preferably being provided by a mobile device communicatively connected to the electronic unit. The status information may be redundant or complementary to primary signals from the injection device that a user may still capture in parallel. In particular, the status information may include a positive confirmation of a dose having been set or corrected, or an indication about a lapse of a minimum holding, delay, or dwell time following completion of a substance expel or piston forwarding activity to inform the user that it is now safe to remove the injection device.

In summary, the present invention concerns an electronic unit in, for, or near an injection device, as part of a medical system for guiding patients through a single injection process and/or throughout a long-term therapy plan. Accordingly, in addition to merely monitor or track a use of the injection device by means of the electronic unit, the system may actively guide patients through individual steps of a handling sequence in real-time, either via basic feedback elements on the injection device or a module incorporating the electronic unit, or via a mobile device communicatively connected to the electronic unit. The guidance may include a motivating element to have patients actually use the injection device and adhere to a prescribed therapy, including gamification aspects and a reward system adapted to the age and preferences of the patient. In other words, the electronic unit flexibly transforms an otherwise mechanical injection device platform into a connected system of seamlessly communicating components that enables a plurality of patient support use cases.

In the present context, the term "injection device" refers to a generally pen-shaped device with an elongate device body defining a longitudinal main device axis. The term "distal end" refers to the end of the injection device where an injection needle is located, the term "proximal end" designates the opposite end thereof.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

BRIEF DESCRIPTION OF THE DRAWINGS

The subj ect-matter of the invention will be explained in more detail in the following text with reference to preferred exemplary embodiments as illustrated in the attached drawings, of which

Fig.1 depicts three variants of a medical injection monitoring and patient support system;
Fig.2 depicts an injection device and a detachable electronic module;
Fig.3 depicts a sequence of operational longitudinal section views of the injection device;
Fig.4 depicts an injection device and a detachable electronic module;
Fig.5 depicts three sample sounds from three distinct injection events;
Fig.6 depicts an injection device with an alternative reverse lock;
Fig.7 is a flowchart illustrating a streamlined method of monitoring an injection process;
Fig.8 depicts an exemplary click reference sound and a model waveform; and
Fig.9 depicts an exemplary click sample sound and three local match measures.

For consistency, the same reference numerals are used to denote similar elements illustrated throughout the drawings.

DETAILED DESCRIPTION OF PREFERRED EMBODIMENTS

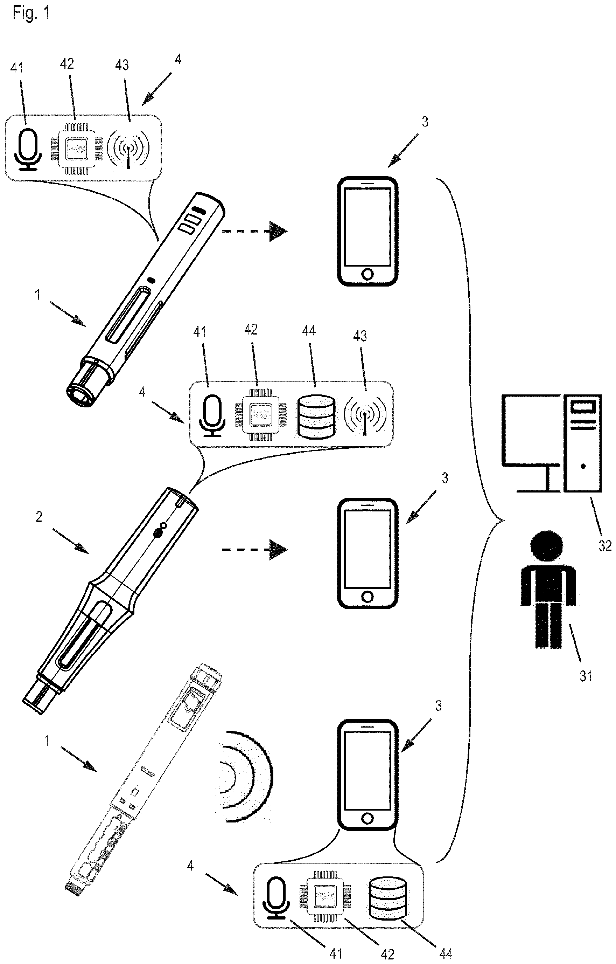

Fig.1 depicts three variants of a medical injection monitoring and patient support system, each variant comprising an injection device 1, an electronic unit 4 and a mobile device 3 such as a smartphone or tablet device running a dedicated application program; or a laptop computer configured accordingly. The mobile device of each variant is adapted to interact with a respective patient 31 as well as a remote server, cloud based computing facility, or expert system 32. In variant one (Fig.1 top), an electronic unit 4 is embedded, or incorporated, in the injection device. In variant two (Fig.1 middle), the electronic unit is part of an electronic module 2 that in turn is detachably mounted on the injection device. In variant three (Fig. 1 bottom) the electronic unit is part of the mobile device placed near the injection device. In variants one and two, the electronic unit comprises a microphone 41, a processing unit 42 and a transmitter unit 43 for wireless transmission of data about the injection progress via Bluetooth Low Energy (BTLE) or equivalent short or near range wireless communication technology to the mobile device as indicated by the dashed horizontal arrows. In variant three the electronic unit includes, or is at least conceptually made up of, specific hardware elements or capabilities of the mobile device. Such elements include a microphone, processing unit, and data storage unit 44, which are shared by the mobile device for the purpose of the present invention. As depicted, the built-in microphone of the mobile device captures sound waves generated by the injection device.

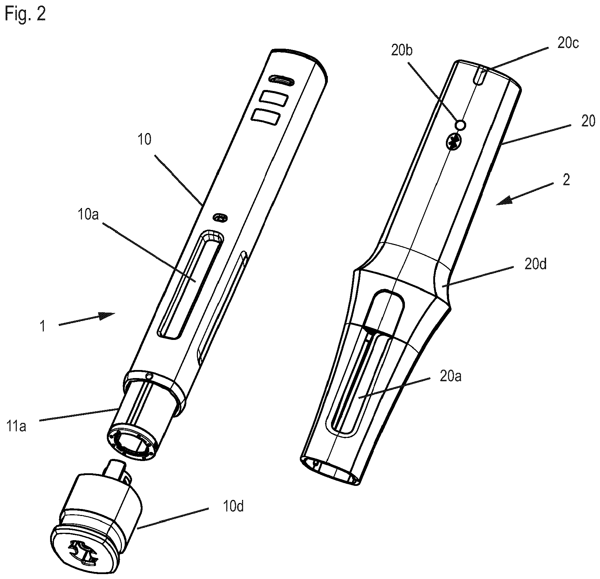

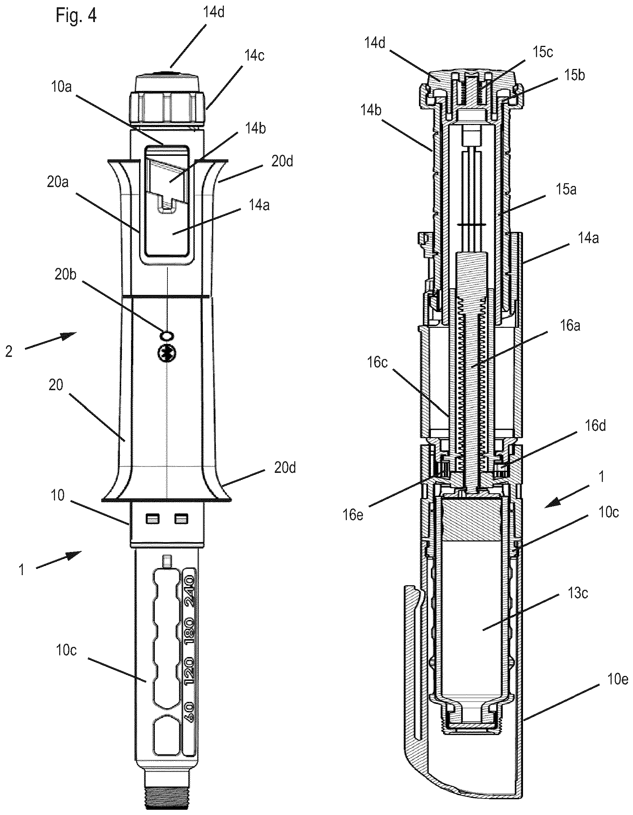

Fig.2 and Fig.4 depict in more detail a first and second embodiment of the specific monitoring system of aforementioned variant two, with the electronic unit comprised in an electronic module 2. The pen-shaped injection device 1 has an elongate housing 10 essentially symmetric around a main axis. The electronic module has an elongate and hollow module housing 20 with a tubular sleeve forming a cavity or inner space adapted to an outer shape of the device housing, such that the injection device may be slidably inserted into the cavity. Included in the module housing is a lock/release mechanism to secure the attachment of the electronic module to the injection device in order to protect against unintended detachment. A detachable, reusable electronic module preferably is adapted for a sustained use during an intended lifetime, with corresponding requirements including a cleanable and waterproof, at least splash water protected, module housing.

The module housing may be ergonomically shaped or designed to accommodate patient-specific usability requirements and to facilitate seizure of the injection device by the patient. The module housing has a cavity adapted to the elongate device housing such that, in the attached state, the electronic module essentially surrounds the injection device over at least a substantial part of the elongate injection device housing, such that the user exclusively seizes and holds the module housing and not the device housing in the attached state. A reliable locking mechanism prevents against axial movement of the module relative to the injection device, specifically during removal of the needle protective cap or the device cap, and during needle insertion. The elongate module housing may be designed to increase usability and provide a better grip by shaping the external surface in a convex manner with distal and/or proximal module housing end sections 20d of increased diameter to facilitate a transfer of axial force from the user to the device. Incidentally, the increased diameter gives rise to some toroidal volume that may be used to accommodate the electronic unit.

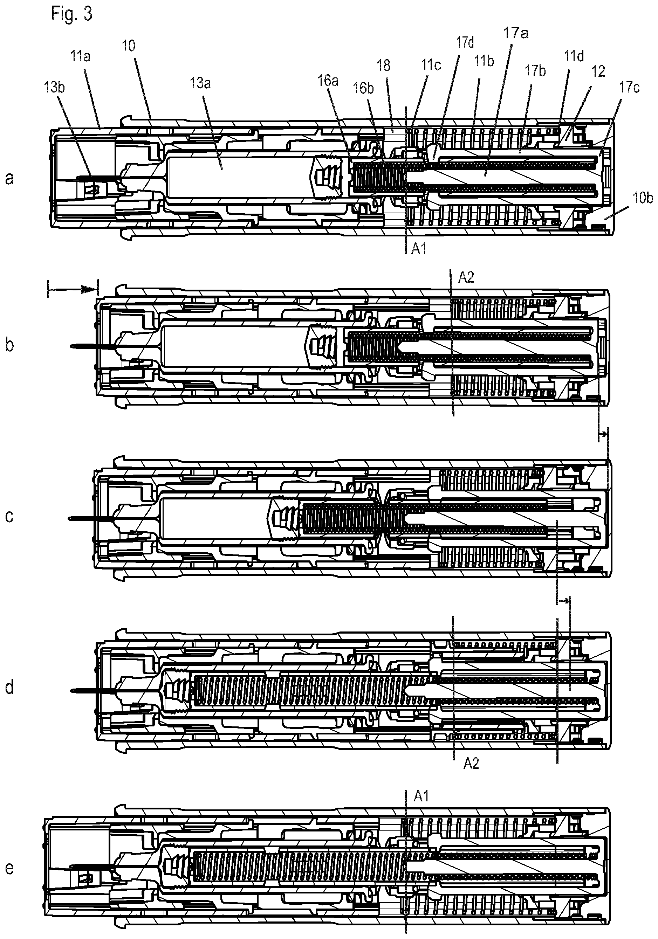

The particular injection device depicted in variant two of Fig.1 and in Fig.2 is an auto-injector as further describe below in connection with Fig.3, comprising a cover sleeve 11a that is movable between a first position (shown in Fig.2) and a second, or retracted, position in which the cover sleeve has moved in a proximal direction to be hidden inside, or enclosed by, the device housing. A needle protective cap 10d mounted onto a distal end of the injection device in order to protect, e.g. during shipping, a needle 13b of the injection device, has to be removed prior to injection, in order to expose the cover sleeve. A syringe 13a is either held immovable in the housing of the auto-injector, or may translate within the housing to insert or retract the needle. The electronic module depicted in Fig.2 further has a rear, or proximal, part where the electronic unit with some or all electronic components as described below is preferably located. Connection and system status indicators 20b, 20c provide visual feedback about a connection status indicative of an established communication link to a mobile device, and about a device, module, or process status including for instance an availability of battery power, a readiness of communication means, an attached/detached status of the electronic module and the injection device, or a progress of an ongoing injection process. The module housing has openings 20a that match with windows 10a of the injection device, which in turn allow for visual inspection of a substance comprised in the injection device.

Fig.3 depicts a sequence 3a to 3e of longitudinal sections of an injection device corresponding to successive operational steps as already described in EP 2781230. The sections depicted are somewhat distorted in a transverse direction for the sake of legibility, and therefore not to scale. The injection device has a device housing 10 closed off by an end cap 10b, and a syringe holder part for accommodating a pre-filled syringe 13a with a needle 13b at a distal end. The injection device also includes a cover sleeve 11a that laterally surrounds the needle in the initial position after removal of a needle protective cap as seen in view 3a and in a final position after removal of the device from the injection site as seen in view 3e. A piston rod 16a transmits force from a drive means onto a piston comprised in the syringe for dispensing or expelling a liquid through the needle. An injection spring 16b serving as drive means and energy storage is pre-tensioned in an initial state between the piston rod 16a and a click pin. The click pin consists of a guide pin 17a arranged within the injection spring, and flexible release snap arms 17b connected to the guide pin via a proximal click pin end surface 17c. The latter also serves as a Start-of-Injection SoI signaling element. In the initial state, inner cams 17d at the distal end of the snap arms engage in indentations or openings of the piston rod to prevent a relative movement of piston rod and click pin. When the injection device is pressed onto an injection site the cover sleeve is axially displaced in proximal direction, i.e. in the direction of the horizontal arrow between views 3a and 3b. This retracting movement of the cover sleeve is transferred to a rigid locking sleeve 18 and further to a linear cover sleeve spring 11b with first and second spring base 11c, 11d, which is thus compressively charged, strained or tensioned. An End-of-Injection EoI signaling element, or end click sleeve 12, is provided adjacent to second cover spring base 11d.

Views 3a to 3e depict in detail the various states of the injection device, starting with an initial state in view 3a in which the cover sleeve spring 11b is least charged or tensioned. View 3b depicts the state after insertion of the needle 13b into the skin of a patient, with the cover sleeve retracted and the cover sleeve spring 11b being compressed accordingly. The compression of the cover sleeve spring is represented by a displacement of a first base 11c of the cover sleeve between a first position (indicated by vertical line A1) and a second position (indicated by vertical line A2). Upon retraction in proximal direction of the cover sleeve 11a and the lock sleeve 18 the cams 17d are released to flex radially outward, following which the entire click pin 17 is accelerated in proximal direction by means of the relaxing injection spring 16. After a short distance indicated by the horizontal arrow between views 3b and 3c, the click pin end surface 17c abuts onto the housing 10 or end cap 10b to generate a SoI click sound. View 3c depicts an ongoing drug expel or piston forward operation driven by the expanding injection spring. In an initial part of the injection spring expansion the end click sleeve 12 is displaced in distal direction by another short distance under the effect of the relaxing injection spring, which in turn causes an additional compressive tensioning of the cover sleeve spring. At the end of the drug expel operation, the end-click sleeve moving is released to move in proximal direction under the force of the expanding cover sleeve spring. After another short distance indicated by the horizontal arrow between views 3c and 3d, the end click sleeve abuts and generates an EoI click sound. After removal of the injection device from the injection site, the cover sleeve and the lock sleeve move in distal or forward direction under the force of the expanding cover sleeve spring. This axial displacement triggers a rotation of the locking sleeve which results in a cam, protrusion, or other guide element of the locking sleeve or the housing to abut and to line up at a step of the housing or of the locking sleeve, respectively. In this final state depicted in view 3e the cover sleeve is locked against further retraction. The abutment of the locking sleeve at the end of the rotation, or at the end of a short linear displacement following the rotation, generates a distinctive lock click sound.

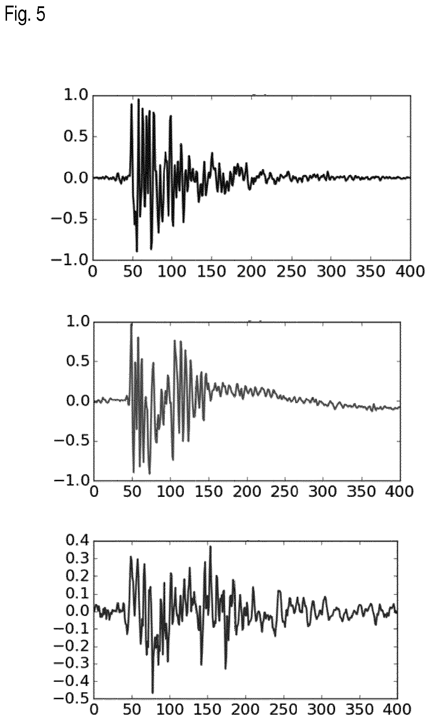

Fig.5 depicts sample sounds recorded with a smartphone from an auto-injector. It is evident that the three distinct injection events give rise to distinct acoustic signatures.

The particular injection device depicted in Fig.4 is a variable dose injection device 1 with a dose dialing facility as amply described for instance in EP 2812055. The housing 10 forms the base of the pen-shaped injection device and is fixed to a carpule holder 10c containing a carpule or cartridge 13c by means of a snap connection. Dosing and delivery components are at least partially arranged in the housing. An indicator sleeve 14a is rigidly inserted into the housing 10 and has an internal thread, to which a thread on an outer surface of a dosing sleeve 14b is engaged. The rotary dosing knob 14c for enabling the user to adjust a dose is arranged on the proximal end of the dosing sleeve. The dosing sleeve features markings in the form of numbers on its outer surface. When the dosing sleeve is screwed out of the housing during the dosing operation, the adjusted dose is displayed in the window 10a. On the proximal end of the injection unit, a discharge button 14d is snapped on the dosing sleeve in such a way that the discharge button can slightly move axially relative to the dosing sleeve and is freely rotatable.

A reusable electronic module 2 has been mounted or fastened to the injection device housing 10 as depicted in the left-hand part of Fig.4. The module housing 20 is designed to be positioned on the injection device housing in such a way as to neither interfere with the dial-and-dose components nor obscure any display window or visual indicator of the device. To this purpose, the module housing has the shape of a tubular sleeve from which the injection device extends at both ends, and has a recess or cut-out 20a that matches with the window 10a. Hence the patient may continue using the injection device in a known manner, despite the presence of the electronic module, with all device interface elements remaining fully accessible throughout the handling sequence. Specifically, in this case the electronic module excludes the presence of a mechanical sensor to mechanically detect a rotation angle or linear shift of a dosing knob or other externally accessible component of the injection device. Likewise, the electronic module excludes the presence of an optical sensor to read a dialed dose from a dialing sleeve.

The right-hand side of Fig.4 shows a longitudinal section through the variable dose injection device in an initial state, after dose selection but prior to removal of a device cap 10e. In order to increase legibility, the section has been drawn with a scale and aspect ratio different from the one in the left-hand drawing. A coupling sleeve 15a is arranged coaxial to the dosing sleeve 14b. Close to its proximal end the coupling sleeve comprises an annular flange 15b that engages, via a distally oriented toothing comprising an exemplary number of twenty teeth evenly distributed about the circumference, with a complementary annular counterface of the dosing sleeve. A dosing click-spring 15c is arranged between the discharge button 14d and the coupling sleeve. Due to the snap-on connection between the discharge button and the dosing sleeve, the annular flange and the counterface are pressed against one another. Turning the dosing knob 14c of the dosing sleeve in a dose-increasing, clockwise dialing direction or in a dose-reducing, counter-clockwise corrective direction relative to the coupling causes the toothing of the counterface to slide over the toothing of the flange, repeatedly performing a slight axial motion back and forth motion that gives rise to a clicking sound and vibration burst at every tooth. The number of clicks is proportionate to the dosage volume, wherein preferably each click or vibration burst corresponds to a single dosage unit, such as an International Unit IU. A relative rotation between the coupling sleeve and the dosing sleeve is inhibited when the discharge button is manually pressed in the distal direction against the spring force.

A sleeve-shaped threaded nut 16c is axially fixed in the housing and rotationally coupled to the coupling sleeve 15a. On its inner side, the threaded nut features a thread that is engaged with the external thread of a piston rod 16a. The piston rod is axially guided by a piston rod guide 10f of the device housing. On a distal end, the threaded nut is provided with a flexible arm 19a having one end solidly anchored in the threaded nut and carrying a tooth, or cam, at a second, free end of the arm. The tooth may radially move or flex in a plane perpendicular to the axis such that the distance between the tooth and the axis varies. The flexible arm is biased radially outward such that the tooth arranged thereon engages with a grating 10g on the inner side of the housing. The flexible arm, the tooth and/or the grating are shaped in such a way that the threaded nut can only rotate in a direction which results in a motion of the piston rod in the distal, dose-expelling direction. Since the threaded nut rotates relative to the housing during the discharge, the tooth of the flexible arm also rotates relative to the grating, which in turn generates a mechanical, i.e. acoustic and tactile discharge feedback signal to the user. A plurality of, such as two or four, flexible arms with corresponding teeth may be provided, wherein the grating is designed such that an exemplary number of twenty time-wise distinct flex-back movements of the flexible arm(s) take place in one turn of the threaded nut. Each flex-back movement generates a mechanical feedback indicative of a dosage unit, or fraction thereof, being expelled, and involves one or several of the flexible arms discharging to abut against a bottom part of the grating.

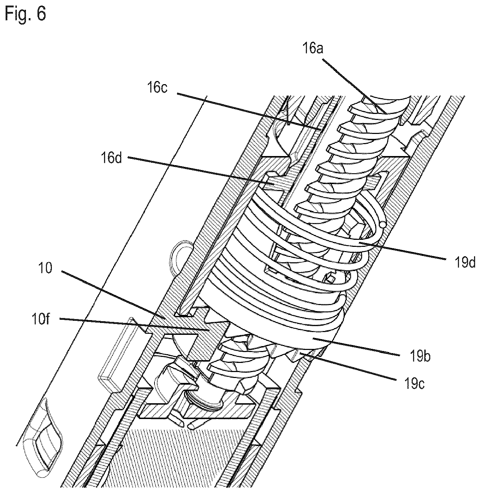

Fig.6 depicts an alternative to the flexible arms of Fig.4 that likewise operates as a reverse lock ensuring that the threaded nut can only be rotated or turned relative to the housing in one direction. On its distal end, the threaded nut 16c is surrounded by an annular toothed disk 19b. The toothed disk can be longitudinally displaced relative to the threaded nut, but is secured against rotating relative to the threaded nut and arranged coaxial thereto. The toothed disk comprises a toothing 19c with an exemplary number of twenty teeth evenly distributed over the circumference and protruding in distal direction. These teeth engage into a counter-toothing on the piston rod guide 10f of the device housing under the effect of a resilient element in the form of a co-axial expel click spring 19d supported on flange 16d of the threaded nut. Upon rotation of the threaded nut and toothed disk the toothing of the disk slides over the counter-toothing of the housing in a combined rotational and axial movement against the force of the expel click spring, repeatedly interrupted by an axial return motion in distal direction. The latter includes a linear acceleration of the toothed disk by the relaxing click spring, until the disk abuts against the counter-toothing to give rise to a click sound and vibration burst. The toothing is advantageously realized asymmetrically such that a relative rotation between the toothed disk and the housing is possible in one direction and blocked in the other direction. The exemplary wedge-shaped toothing visible in Fig.3 translates the rotation of the toothed disk into a combined axial and rotational movement, with tangential force components leading to a torque on the device housing that may be determined and exploited independently from the axial injection force component.

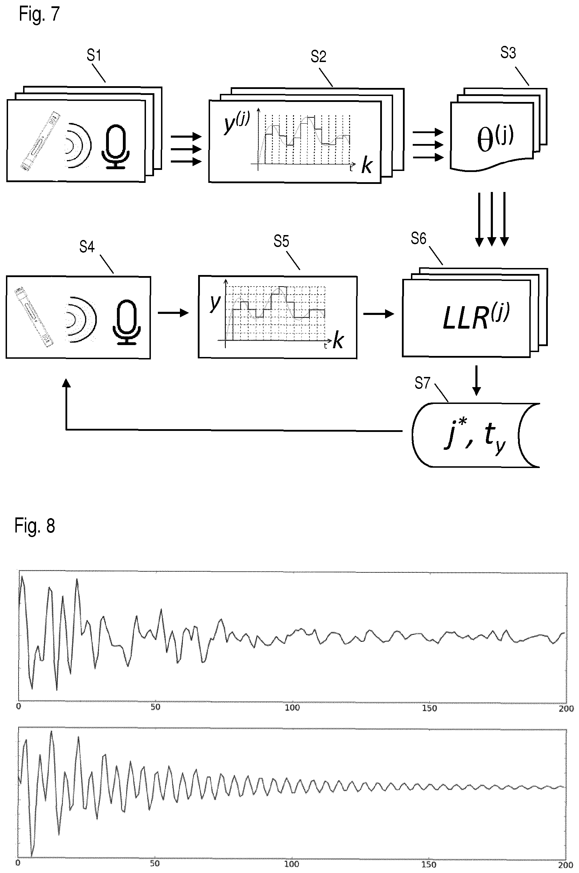

Fig.7 is a flowchart illustrating a streamlined method of monitoring an injection process according to the invention. In step S1, exemplary first, second, and third reference sounds $y^{(j)}$ ($j$ = 1...3) respectively characteristic of a first, second, and third injection event (index $j$) occurring during reference execution of the injection process, are recorded. It is apparent that the number of injection events considered may be any number larger than one, and preferably equals two, three, or four. In step S2, the reference sounds are sampled at an exemplary sampling frequency of 44.1 kHz to generate a discrete-time signal of length $K$ as a data set with an exemplary number of $K$=200 data points. In step S3, a small number $N^{(j)}$ of model parameters $\theta^{(j)}$ are determined for each of the reference sounds as detailed in the following, and stored for subsequent repeated use in a memory of the electronic unit. A representative number of, for instance, ten to twenty reference sound recordings per injection event may be exploited in order to obtain model parameters incorporating a minimum unavoidable variance in click sound generation. The preparatory steps may be executed in a controlled environment with minimal background noise, with specialized equipment or with the electronic unit that will be subsequently used during the recurring tracking steps. Updates of the model parameters may be performed as needed and deemed suitable. Alternatively or additionally, model parameters may be synthesized, i.e. derived from simulated operation of the sound generating means with known or assumed elastic properties, or from other heuristic methods.

In recurring steps S4 to S7, during the injection process to be monitored, sample sounds $y$ originating from a-priori unidentified device events are recorded, sampled, and temporarily stored in a memory of the electronic unit, steps S4 and S5. In step S6, a first, second, and third match measure $LLR^{(j)}$ indicative of a match of the sample sound with the first, second, and third reference sound, respectively, is determined, based solely on the three sets of model parameters $\theta^{(j)}$ and the most recent sample sound data file $y$. The three match measures are determined at a same time, possibly one after the other and hence not necessarily strictly in parallel, such that they are ultimately available for relative comparison. From the three match measures, a largest or otherwise preferred one is identified, and the index $j^*$ of the corresponding injection event is stored as a corresponding identifier together with a timestamp $t_y$ of the sample sound in a memory of the electronic unit in step S7.

In some more detail the method of monitoring an injection process executed by means of an injection device comprises the steps of

- recording a first and a second reference sound to generate discrete-time signals and storing the latter as a first and second reference file or parameter set, respectively characteristic of a first and a second reference injection event type or class, wherein instances of these event types occur sequentially during any injection process and individually generate click sounds,
- recording, during the injection process to be monitored, a sample sound originating from a sample event,
- determining a first and a second match measure indicative of a match, i.e. a probability of common event origin of the sample sound and the first and the second reference sound, respectively;
- identifying the sample event as being of the first reference injection event type if the first match measure exceeds the second match measure, and vice versa;
- storing a timestamp of the recording together with an identifier of the first or second event type as which the sample event has been identified.

An exemplary method of identifying injection events by means of their acoustic signature is based on a Linear State Space (LSS) Model approach. The recorded audio reference sounds are interpreted as the output of a Linear State Space Model with a few parameters that have to be estimated. The recorded audio reference sound as a discrete-time signal $y = (y_1, ..., y_K)$ of length K is to be explained as the output of the $n$-dimensional linear state-space model

$$X_k = AX_{k-1} + \varepsilon_k$$

$$y_k = CX_k + Z_k$$

where $X_k$ are states, $A$ is the n×n state (or system-) matrix, $C$ is the 1×n vector that maps states to the output, $\varepsilon_k$ denotes state noise and $Z_k$ denotes observation noise, both characterized by their respective variance $\sigma_\varepsilon^2$, $\sigma_Z^2$. The model dimension n is a parameter that may be tuned in the algorithm. The model parameters $\theta = (A, \sigma_\varepsilon^2, \sigma_Z^2)$ are unknown and have to be estimated. For that purpose, an Expectation Maximization (EM) algorithm may be employed, which can be efficiently executed by modelling the LSSM as a factor graph as described in a paper by H.-A. Loeliger et al, "The factor graph approach to model-based signal processing," Proceedings of the IEEE vol. 95, no. 6, pp. 1295-1322, 2007.

The state matrix $A$ is constrained to have block-Jordan form, i.e. its only non-zero entries are the $2 \times 2$-Jordan-blocks on the diagonal. The Jordan-blocks are further constrained to be scaled rotation matrices:

$$A = \begin{pmatrix} A_1 & 0 & 0 \\ 0 & A_2 & 0 \\ 0 & 0 & \ddots \end{pmatrix}$$

where

$$A_i = \rho_i \begin{pmatrix} \cos(\omega_i) & -\sin(\omega_i) \\ \sin(\omega_i) & \cos(\omega_i) \end{pmatrix}$$

with $\rho_i$ and $\omega_i$ being the decay factor and frequency of the $i^{th}$ Jordan-block. The appropriate state-output vector $C$ is given by $C = (1\ 0\ 1\ 0 ...)$. The decay factors of each state matrix may be scaled, in particular increased by a common factor, in order to improve the approximation of the original reference sound data.

Fig.8 depicts an exemplary click reference sound (top), and a model waveform (bottom) reconstructed from the model parameters of a Linear State Space (LSS) Model as estimated from the original reference sound.

The result of the model estimation for a reference click sound characterizing the $j^{th}$ injection event are the parameter sets $\theta^{(j)} = (A^{(j)}, \sigma_\varepsilon^{2,(j)}, \sigma_Z^{2,(j)})$, each comprising a number $N^{(j)}$ of model parameters. The algorithm for identifying an injection event from a sample sound audio file $y = (y1, ..., y_K)$ includes a likelihood filter which calculates the Log-Likelihood Ratios $LLR_k^{(j)}$ exclusively from the parameter set $\theta^{(j)}$ and the sample sound data $y$ as follows:

$$LLR_k^{(j)} = \log\left( \frac{\max\limits_{X_0} p(y|\theta^{(j)}, X_0)}{p(y|\theta^{(j)}, X_0 = 0)} \right)$$

This quantity is the ratio of the likelihood of the hypothesis that the injection event $j$ as represented by its reference sound parameters $\theta^{(j)}$ is present in, or at the origin of, the sample sound $y$ at time $k$ and the likelihood of the hypothesis that there is only noise in the sample sound signal. The algorithm determines a maximum $LLR^{*(j)}$ at max-time $k^{*(j)}$ for each of at least two candidate injection events $j$, and identifies the injection event with the highest maximum $LLR^*$ as the true originator of the sample sound being investigated. A corresponding identifier $j^*$ together with a timestamp $t_y$ of the sample sound and optionally the highest maximum LLR* are retained for further evaluation or transmission, while the sample sound data is discarded.

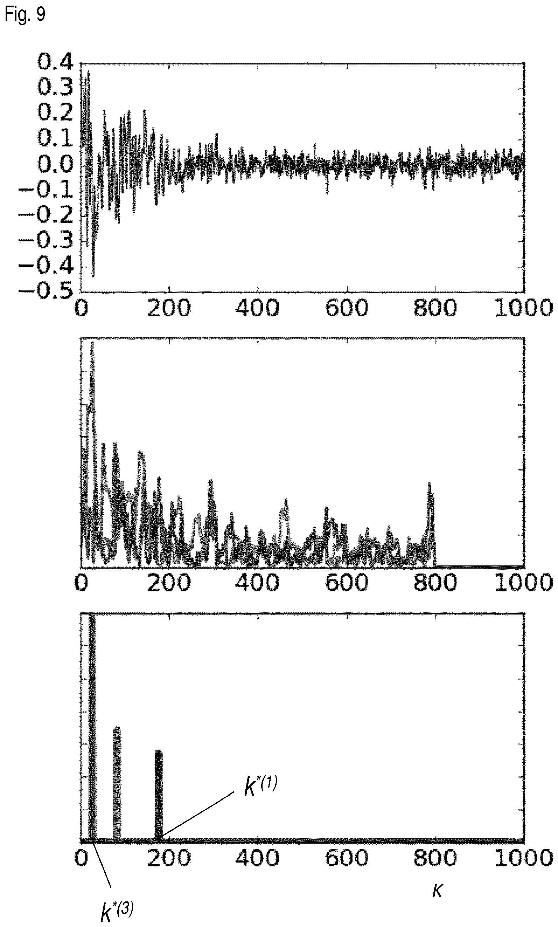

Fig.9 depicts an exemplary click sample sound (top), the corresponding LLR for each of three distinct candidate events, calculated for each time $k$ from $k=1$ to $k=800$ (middle), and the LLR peak values occurring at max-time $k^{*(1)}$, $k^{*(2)}$ and $k^{*(3)}$ (bottom). The maximum LLR of injection event No. 3, a cover sleeve locking event, is preponderant and the device event at the origin of the sample sound thus identified as a cover sleeve locking.

The number $N^{(j)}$ of the above model parameters depends on, or includes, the dimension or rank of the LSS model and of the corresponding state matrix $A^{(j)}$. This number does not have to be identical, for the purpose of the subsequent recurring calculation steps, for all injection events considered, and may in fact be chosen dependent on the injection event. Restricting the number of model parameters to an optimized minimum reduces storage and processing power requirements. By way of example, a pre-determined number of Jordan blocks and/or corresponding frequencies $\omega_i$ are estimated initially from the reference sound based on established model estimation techniques. Subsequently, the estimated frequencies are analyzed, and a subset thereof is selected. In particular, the frequencies coinciding with peaks of a Discrete Fourier Transform (DFT) of the reference sound, or otherwise dominant frequencies may be chosen.

In variants with the electronic unit being integrated in the injection device or being part of an electronic module (variant one and variant two of Fig. 1), the dedicated electronic unit includes a microphone, a processor unit including microprocessor or microcontroller elements suitably configured and physically mounted on Printed Circuit Boards, an antenna for wireless communication preferably according to the Bluetooth Low Energy (BLE) standard, and a power source or energy storage such as a battery, with all constituents being communicatively interconnected as needed via wired connections. The microprocessor units may include an analog-to-digital converter for preparing a digital sensor output signal, an evaluation unit for evaluating and consolidating sensor output signals from different sensors and/or with distinct time-stamps and for preparing consolidated information, a data storage or event recording unit for storing dynamic injection process information as well as unique identification data of a user, and a communication unit for transmitting and receiving wireless signals via the antenna.

The wireless communication between the electronic unit and the mobile device may be subject to several features of the Bluetooth LE Core Specification that cover the encryption, trust, data integrity and privacy of the user's data. These features include advanced out-of-band pairing where the electronic unit and the mobile device involved in the communication exchange their identity information to set up trust and get the encryption keys ready for the future data exchange, or the ability to send authenticated data over an unencrypted transport between two devices with a trusted relationship.

The electronic unit may also include a timer and/or real time clock to provide absolute or relative clock information as needed for time-stamping of injection events or timing of a thermal equilibration process following retrieval of the injection device from a refrigerator. The power source or energy storage may be rechargeable, and to that end may include a USB connector, or be adapted for wireless power transfer from a docking station. The docking station may accommodate the injection device, the electronic module, or the injection device together with the attached electronic module.

The electronic unit, specifically for use in conjunction with an injection device for repeated delivery of small amounts of a drug from a container that is to be stored in a cooled place between the injections, is itself adapted to be stored in a refrigerator at an exemplary cooling temperature of between 2°C and 12°C. The electronic unit may then be activated from stand-by upon removal from the refrigerator, e.g. by measuring a temperature exceeding a limit temperature. The electronic module may alternatively be activated or woken up from an idle state by a passive switching means responsive to a sustained change in temperature or illumination to which the injection device or the electronic module is exposed upon removal from the fridge. The passive switching means does not require any energy while placed in the refrigerator, and thus contributes to extended battery use. For instance, a shape memory alloy based electrical contact switch that is activated at a threshold temperature between the temperature of the refrigerator and room temperature may be used, or a photovoltaic cell designed to activate the processing unit and battery power supply upon exposure to daylight.

The electronic unit may be equipped with, or connected to, additional reading units, such as an optical barcode reader for one or two-dimensional (QR) barcodes, or an electromagnetic Radio-Frequency IDentification (RFID) reader for identifying passive RFID tags containing electronically stored information. The barcode or RFID tag is attached to a container such as a syringe or carpule having been or being inserted in the injection device, and representing a lot or batch number or otherwise indicative of a drug comprised in the container. An electronic unit as part of a detachable electronic module may also employ the barcode or RFID reader to identify the injection device to which the module has been or is being attached. Alternatively, the electronic unit of the module may include a Near-Field Communication (NFC) unit for receiving an identifier or serial number transmitted by the injection device. Accordingly, drug containers being exchanged in reusable injection devices and transfer of the electronic module to another disposable injection device may be detected. In the latter case, a processing unit adapted to receive an identification of the injection device, either manually from a user or via the aforementioned reading units, may proceed to selecting the corresponding reference sounds or model parameters from a multi-injection-device reference sound collection or storage.

In case the mobile device is within reach of the user and communicatively connected, at the time of injection, to the electronic unit of the injection device or the electronic module, data upload may occur instantaneously and individually after each identified injection event. Data upload may also occur only following injection process completion and include consolidated information about the entire injection process. With the mobile device being placed within visual and/or audible reach of the user, it may provide real-time Instructions for Use, guiding the user through the successive steps of a single injection event, and providing additional imminent feedback based on historical information. Visually impaired users may benefit from a kind of enlarged display enabled by the mobile device when duplicating information of a display of the mobile device.

In case no mobile device is available at the time of injection, data upload is postponed, and real-time user support exclusively provided by means of the visual, audible and/or tactile status indicators of the electronic unit. Relevant data is locally stored in a data memory of the electronic unit until data upload to the mobile device is possible, which may be the case only at a next injection, and which may occur both in an attached or detached state of the electronic module and the injection device. Received injection data may be further conveyed from the mobile device to external expert systems in order to individually assist patients, relatives, caregivers and healthcare professionals with customized information, and with a goal to improve patient adherence to a prescribed therapy plan. Accordingly, the mobile device not only acts as a user interface providing information to a user that mirrors or complements the status information generated by the electronic device itself, but also acts as a gateway to a cloud-based and secure data server and data evaluation facility. Alternatively, and particularly in case of an extended absence or unavailability of the mobile device, the data may be uploaded directly to a stationary receiver, for evaluation by an expert system and/or storage in a cloud computing environment. Such upload may take place by incorporating standard cellular mobile phone, e.g. GSM, or forthcoming low-power long range wireless communication technologies into the electronic module.

Exemplary data recorded by the electronic module and subsequently or occasionally transmitted to the mobile device for storage or further dissemination includes at least a timing and a quality information of an injection event. Basic timing information such as time and date may be complemented by an indication of the injection site, i.e. to which body part of the patient the drug has been injected. To that purpose, the mobile device may accept manually entering the injection site information, possibly along with a self-appraisal of patient well-being or therapy outcome. Basic quality information includes an indication whether the injection was performed correctly or whether a deviation occurred from the correct intended usage. In the latter case, the quality information preferably comprises an indication about the deviation observed, including an interruption of the injection as caused by removal of the injection device from the injection site during injection, or a short holding time as caused by removal of the injection device from the injection site before lapse of a holding time after drug expelling. ICT related information such as a network address or identifier of the injection device, the electronic module, and/or the mobile device as well as information on the communication network(s) employed by the mobile device may be appended to the injection data at any stage of the transmission.

The mobile device has to be set up initially by installing and configuring a smartphone application program and by duly identifying and enrolling the user, such as by means of a patient onboarding card conveying, by means of NFC or optical barcodes, all relevant data to the mobile device. The mobile device may then provide assistance, support and/or guidance to the user, such as advising about counter measures in case a deviation is detected, informing the user about drug expiration dates, and providing interactive and/or animated instructions for use corresponding to all stages of the injection process. The complete injection process support may commence with a reminder function supporting the user to remember when to perform an injection according to a prescribed therapy plan, continue with the timing of a thermal equilibration process following retrieval of the injection device from a refrigerator, a provision of instructions for attaching the electronic module and removing a needle protective cap from the injection device, suggesting an injection site e.g. different from the last injection site, monitoring the drug administration, and end with a provision of instructions for detachment of the electronic module and disposal of the injection device. The user may also be alerted in case a residual volume of drug decreases below a threshold. The electronic unit or the mobile device may also notify the user about an immediately preceding inactivity shut-down of the unit, together with a warning that this may have led to the last injection process not having been registered correctly. The mobile device may also store the data transmitted by the electronic unit, specifically timing and a quality information of a sequence of injection events as a consolidated injection history, and occasionally forward this data to the optional remote server, cloud based computing facility, or expert system.

In the context of healthcare data management, it is advisable to treat monitoring data that has been collected by any kind of smart monitoring unit, including but not limited to the electronic unit according to the present invention, as Protected Health Information (PHI). In order to avoid data abuse, misappropriation, manipulation or the like to the detriment of the patient or a healthcare provider, several measures may be envisaged. In the following patient adherence data is referred to as the monitoring data accumulated by the electronic unit, including records of time-stamped injection events, but possibly including a log or transcript of a bidirectional interaction between patient and electronic device, including in particular any feedback or recommendation provided to the user. Communication of such patient adherence data from a mobile device or end-user gateway of the user to a remote site has to guarantee privacy and integrity of the data. Specifically, communications containing PHI or equivalent patient adherence data and transmitted electronically over open networks have to be encrypted and protected from being intercepted by anyone other than the intended recipient. Likewise, information technology systems receiving PHI must be protected from intrusion, and the identity of the intended recipient must be authenticated. Data integrity on the other hand may be increased by means of check sum, double-keying, message authentication, and digital signature.

One option for storing patient adherence data in a computing cloud or other distributed data server structure comprises storing the data, on the one hand, in an encrypted format that restricts access to a holder of a particular key. On the other hand, data may be stored as anonymized cloud data, such that the patient may not be identified from the data. Anonymizing data may include location fuzzing, noise addition, permutation, k-anonymization, 1-diversity, or similar, and enable analysis of aggregated anonymized but otherwise unencrypted data by research institutions or health care stakeholders. In addition, personally identifiable patient adherence data may be shared directly with a trusted named doctor using standard public-key cryptography.

Another option for storing patient adherence data in a computing cloud comprises uploading personally identifiable patient adherence data to the cloud where it is stored. Data transfer over networks is fully encrypted, and the stored data is accessible for detailed analysis by trusted experts (e.g., cloud-based doctor analysis). This option provides maximum flexibility with regards to analysis of personal data, and enables patients to use a web-based interface to access their data.

A third option enabling a specialized healthcare data service provider to analyze PHI without violating the privacy of the patient resides on encrypting the patient adherence data according to a homomorphic encryption scheme, and sending the encrypted data to a data service provider. The latter may then evaluate the encrypted data and derive, or calculate, encrypted statistical or otherwise augmented information there from. The encrypted information is sent back to a trusted doctor of the patient, or to the end-user gateway embodying some medical expert knowledge, where it is decrypted and used for a refined diagnosis and/or adapted therapy. Fully homomorphic encryption schemes are compatible with an arbitrary number of additions and multiplications on encrypted data. Thus, all data processing functions comprising polynomial operations, including statistical functions such as the mean, standard deviation and logistical regressions, are compatible with homomorphic encryption schemes, and may be utilized by the healthcare data service provider.

While the invention has been described in detail in the drawings and foregoing description, such description is to be considered illustrative or exemplary and not restrictive. Variations to the disclosed embodiments can be understood and effected by those skilled in the art and practicing the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims shall not preclude the existence of further meaningful combinations of these elements or steps.

LIST OF REFERENCE NUMERALS

| | |
|---|---|
| 1 | injection device |
| 10 | device housing |
| 10a | window |
| 10b | end cap |
| 10c | carpule holder |
| 10d | needle protective cap |
| 10e | device cap |
| 10f | piston rod guide |
| 10g | grating |
| 11a | cover sleeve |
| 11b | cover sleeve spring |
| 11c, 11d | first, second cover sleeve spring base |
| 12 | end-click sleeve |
| 13a | syringe |
| 13b | needle |
| 13c | carpule |
| 14a | indicator sleeve |
| 14b | dosing sleeve |
| 14c | dosing knob |
| 14d | discharge button |
| 15a | coupling sleeve |

| 15b | annular flange |
| --- | --- |
| 15c | dosing click-spring |
| 16a | piston rod |
| 16b | injection spring |
| 16c | threaded nut |
| 16d | flange |
| 17a | guide pin |
| 17b | snap arm |
| 17c | click pin end surface |
| 17d | cam |
| 18 | locking sleeve |
| 19a | flexible arm |
| 19b | toothed disk |
| 19c | toothing |
| 19d | expel click-spring |
| 2 | electronic module |
| 20 | module housing |
| 20a | opening |
| 20b | connection status indicator |
| 20c | system status indicator |
| 20d | module housing end section |
| 3 | mobile device |
| 31 | patient |
| 32 | data server |
| 4 | electronic unit |
| 41 | microphone |
| 42 | processing unit |
| 43 | transmitter unit |
| 44 | data storage unit |

**Claims**

1. A method of monitoring an injection process executed by means of an injection device (1), comprising

   - recording a first and a second reference sound $y^{(j)}$ respectively characteristic of a first and a second injection event $j$ sequentially occurring during reference execution of the injection process;
   - recording, by a microphone (41) and a processing unit (42) connected to the microphone, during the injection process to be monitored, a sample sound $y$ originating from a device event, and generating a timestamp indicative of a time of recording of the sample sound;
   - determining a first and a second match measure linear likelihood ratio $LLR^{(j)}$ indicative of a match of the sample sound with the first and the second reference sound, respectively;
   - identifying the device event as a first injection event if the first match measure exceeds the second match measure, and identifying the device event as a second injection event if the second match measure exceeds the first match measure;
   - storing the timestamp together with an identifier $j^*$ of the identified injection event.

2. The method of claim 1, comprising,

   - identifying, from the first and second reference sound, a first and second set $\theta^{(j)}$ of respective model parameters, and
   - determining the first and second match measure based on the first and the second set of model parameters, respectively.

3. The method of claim 2, wherein a number $N^{(1)}$ of parameters in the first set of model parameters is different from a number $N^{(2)}$ of parameters in the second set.

4. The method of claim 2, comprising

- generating, from the sample sound, a discrete-time signal y with a number $K$ of data points, each data point comprising time $k$ and corresponding data $y_k$;
- determining, for each data point of the discrete-time signal, a local match measure between the corresponding data $y_k$ of the discrete-time signal and an evaluation of the first set $\theta^{(1)}$ of model parameters at time $k$, and
- determining the first match measure as the maximum local match measure.

5. The method of claim 2, wherein the model parameters include a frequency $\omega_i$ and a decay factor $\rho_i$ of a scaled rotation matrix A of a Linear State Space Model of the first and second reference sound.

6. The method of one of claims 1 to 5, wherein the first injection event is a start of a drug expel process signaled by a Start-of-Injection SoI click and the second injection event is an end of the drug expel process signaled by an End-of-Injection EoI click, the method further comprising

- identifying the device event as a start of a drug expel process,
- recording, subsequently, a further sample sound originating from a further device event, generating a timestamp indicative of a time of recording of the further sample sound, and identifying the further device event as an end of the drug expel process,;
- evaluating a time delay between the timestamps of the end and of the start of the drug expel process to conclude on a successful drug expel process execution.

7. The method of claim 6, wherein the injection device is an auto-injector with an injection spring for expelling a fixed dose of drug from a syringe (13a) included in the injection device, and with a cover sleeve (11a) biased by a cover sleeve spring for protecting a needle of the syringe, the method comprising

- recording a third reference sound characteristic of the cover sleeve being locked in a needle protective position;
- recording, by the microphone and the processing unit, during the injection process to be monitored, a next sample sound originating from a next device event, and generating a timestamp indicative of a time of recording of the next sample sound;
- determining a first, second, and third match measure indicative of a match of the next sample sound with the first, second, and third reference sound, respectively;
- identifying the next device event as the cover sleeve locking in a protective position if the third match measure exceeds the first and the second match measure,
- evaluating a time delay between the timestamp of the cover sleeve locking event and a timestamp of the end of the drug expel process to conclude on a sufficient holding time.

8. The method of one of claims 1 to 5, wherein the injection device is a variable dose injection device generating dialing and expel clicks, wherein the first injection event is a dosage unit setting event signaled by a dialing click, and the second injection event is a dosage unit expelling event signaled by an expel click, the method comprising

- identifying each of a series of device events as a dosage unit expelling event,
- counting the number of dosage unit expelling events in the series to determine a expelled dose.

9. The method of one of claims 6 to 8, comprising

- deriving, from a plurality of stored time stamps and injection event identifiers, consolidated injection information about the injection process including a quality information of the injection process, and
- transmitting, by a wireless communication unit (43) connected to the processing unit, the consolidated injection information to a mobile device (31).

10. An electronic unit comprising a microphone (41) and a processing unit (42) connected to the microphone, adapted to cooperate with an injection device generating a click sound during an injection process, wherein the unit is configured to

- record and time-stamp, by the microphone and the processing unit, during the injection process to be monitored, a sample sound y originating from a device event,
- determine a first and a second match measure linear likelihood ratio $LLR^{(j)}$ indicative of a match of the sample sound with a first and a second reference sound $y^{(j)}$ respectively characteristic of a first and a second injection event $j$ sequentially occurring during a reference execution of the injection process, from a respective set of model parameters stored in the electronic unit and identified from the corresponding reference sound;
- identify the device event as a first event if the first match measure exceeds the second match measure, and vice versa;
- store the timestamp together with an identifier of the identified event.

11. The electronic unit of claim 10, **characterized in that** the unit is embedded in a mobile device, and **in that** the microphone and processing unit are shared with the mobile device, wherein the mobile device may be placed in the vicinity of the injection device for monitoring the injection process.

12. The electronic unit of claim 10, **characterized in that** the unit is integrated into the injection device or into an electronic module with a module housing adapted to be removably attached to the injection device, and **in that** it comprises two microphones.

13. The electronic unit of claim 10, **characterized in that** the unit is integrated into the injection device or into an electronic module with a module housing adapted to be removably attached to the injection device, and **in that** it comprises a status indicator (20c) for indicating a status of the system to a user, in particular for indicating a lapse of a holding time.

14. The electronic unit of claim 10, **characterized in that** the unit is integrated into the injection device or into an electronic module with a module housing adapted to be removably attached to the injection device, **characterized in that** it comprises a data storage unit (44) for storing at least a timing and a quality information of an injection event, and a wireless communication unit (43) for transmitting the stored information to a mobile device (3).

15. A computer program which, when being executed by a processing unit of a mobile device for monitoring an injection process performed by means of an injection device, causes the processing unit to operate according to one of claims 1 to 8.

**Patentansprüche**

1. Verfahren zum Überwachen eines Injektionsprozesses, der mittels einer Injektionsvorrichtung (1) ausgeführt wird, umfassend

- Aufzeichnen eines ersten und eines zweiten Referenztons $y^{(j)}$, die jeweils kennzeichnend für ein erstes und ein zweites Injektionsereignis $j$ sind, die während der Referenzausführung des Injektionsprozesses nacheinander auftreten;
- Aufzeichnen, durch ein Mikrofon (41) und einer Prozesseinheit (42), die mit dem Mikrofon verbunden sind, während des zu überwachenden Injektionsprozesses, eines Probetons $y$, der von einem Vorrichtungsereignis stammt, und Erzeugen eines Zeitstempels, der bezeichnend für eine Zeit der Aufzeichnung des Probetons ist;
- Bestimmen eines ersten und eines zweiten linearen Messübereinstimmungswahrscheinlichkeitsverhältnisses $LLR^{(j)}$, das bezeichnend für eine Übereinstimmung des Probetons mit dem ersten beziehungsweise dem zweiten Referenzton ist;
- Identifizieren des Vorrichtungsereignisses als ein erstes Injektionsereignis, falls die erste Messübereinstimmung die zweite Messübereinstimmung überschreitet, und Identifizieren des Vorrichtungsereignisses als ein zweites Injektionsereignis, falls die zweite Messübereinstimmung die erste Messübereinstimmung überschreitet;
- Speichern des Zeitstempels zusammen mit einer Bezeichnung $j^*$ des identifizierten Injektionsereignisses.

2. Verfahren nach Anspruch 1, umfassend,

- Identifizieren, aus dem ersten und dem zweiten Referenzton, eines ersten und zweiten Satzes $\theta^{(j)}$ jeweiliger Modellparameter, und
- Bestimmen der ersten und der zweiten Messübereinstimmung basierend auf dem ersten beziehungsweise dem zweiten Satz von Modellparametern.

3. Verfahren nach Anspruch 2, wobei sich eine Anzahl $N^{(1)}$ von Parametern in dem ersten Satz von Modellparametern von einer Anzahl $N^{(2)}$ von Parametern in dem zweiten Satz unterscheidet.

4. Verfahren nach Anspruch 2, umfassend:

- Erzeugen, aus dem Probeton, eines zeitdiskreten Signals $y$ mit einer Anzahl $K$ von Datenpunkten, jeder Datenpunkt umfassend die Zeit k und entsprechende Daten $y_k$;
- Bestimmen, für jeden Datenpunkt des zeitdiskreten Signals, einer lokalen Messübereinstimmung zwischen den entsprechenden Daten $y_k$ des zeitdiskreten Signals und einer Bewertung des ersten Satzes $\theta^{(l)}$ von Modellparametern zu der Zeit $k$, und
- Bestimmen der ersten Messübereinstimmung als die maximale lokale Messübereinstimmung.

5. Verfahren nach Anspruch 2, wobei die Modellparameter eine Frequenz $\omega_i$ und einen Abklingfaktor $\rho_i$ einer skalierten Rotationsmatrix $A$ eines linearen Zustandsraummodells des ersten und des zweiten Referenztons einschließen.

6. Verfahren nach einem der Ansprüche 1 bis 5, wobei das erste Injektionsereignis ein Beginn eines Arzneimittelausstoßprozesses ist, der durch einen Beginn-der-Injektion-Klick (Sol-Klick) signalisiert wird, und das zweite Injektionsereignis ein Ende des Arzneimittelausstoßprozesses ist, das durch einen Ende-der-Injektion-Klick (Eol-Klick) signalisiert wird, das Verfahren ferner umfassend:

- Identifizieren des Vorrichtungsereignisses als ein Beginn eines Arzneimittelausstoßprozesses,
- anschließendes Aufzeichnen eines weiteren Probetons, der von einem weiteren Vorrichtungsereignis stammt, Erzeugen eines Zeitstempels, der bezeichnend für ein Zeit der Aufzeichnung des weiteren Probetons ist, und Identifizieren des weiteren Vorrichtungsereignisses als ein Ende des Arzneimittelausstoßprozesses;
- Bewerten einer Zeitverzögerung zwischen den Zeitstempeln des Endes und des Beginns des Arzneimittelausstoßprozesses, um auf eine erfolgreiche Arzneimittelausstoßprozessausführung zu schließen.

7. Verfahren nach Anspruch 6, wobei die Injektionsvorrichtung ein Autoinjektor mit einer Injektionsfeder für eine Ausstoßung einer festen Arzneimitteldosis aus einer Spritze (13a) ist, die in der Injektionsvorrichtung eingeschlossen ist, und mit einer Abdeckhülse (11a), die durch eine Abdeckhülsenfeder zum Schützen einer Nadel der Spritze vorgespannt ist, das Verfahren umfassend:

- Aufzeichnen eines dritten Referenztonkennzeichens der Abdeckhülse, die in einer Nadelschutzposition verriegelt ist;
- Aufzeichnen, durch das Mikrofon und der Prozesseinheit, während des zu überwachenden Injektionsprozesses, eines nächsten Probetons, der von einem nächsten Vorrichtungsereignis stammt, und Erzeugen eines Zeitstempels, der bezeichnend für eine Zeit der Aufzeichnung des nächsten Probetons ist;
- Bestimmen einer ersten, zweiten und dritten Messübereinstimmung, die bezeichnend für eine Übereinstimmung des nächsten Probetons mit dem ersten, zweiten beziehungsweise dritten Referenzton ist;
- Identifizieren des nächsten Vorrichtungsereignisses als die Abdeckhülsenverriegelung in einer Schutzposition, falls die dritte Messübereinstimmung die erste und die zweite Messübereinstimmung überschreitet,
- Bewerten einer Zeitverzögerung zwischen dem Zeitstempel des Abdeckhülsenverriegelungsereignisses und einem Zeitstempel des Endes des Arzneimittelausstoßprozesses, um auf eine ausreichende Haltezeit zu schließen.

8. Verfahren nach einem der Ansprüche 1 bis 5, wobei die Injektionsvorrichtung eine variable Dosierinjektionsvorrichtung ist, die Wähl- und Ausstoßklicks erzeugt, wobei das erste Injektionsereignis ein Dosiseinstellungsereignis ist, das durch einen Wählklick signalisiert wird, und das zweite Injektionsereignis ein Dosiseinheitsausstoßungsereignis ist, das durch einen Ausstoßklick signalisiert wird, das Verfahren umfassend:

- Identifizieren jedes einer Reihe von Vorrichtungsereignissen als ein Dosiseinheitsausstoßungsereignis,
- Zählen der Anzahl der Dosiseinheitsausstoßungsereignisse, in der Reihe, um eine ausgestoßene Dosierung zu bestimmen.

9. Verfahren nach einem der Ansprüche 6 bis 8, umfassend:

- Ableiten, aus einer Vielzahl von gespeicherten Zeitstempeln und Injektionsereignisbezeichnern, von konsolidierten Injektionsinformationen über den Injektionsprozess, einschließlich Qualitätsinformationen des Injektionsprozesses, und
- Übertragen, durch eine drahtlose Kommunikationseinheit (43), die mit der Prozesseinheit verbunden ist, der konsolidierten Injektionsinformationen an eine mobile Vorrichtung (31).

**10.** Elektronische Einheit, umfassend ein Mikrofon (41) und eine Prozesseinheit (42), die mit dem Mikrofon verbunden ist, die angepasst ist, um mit einer Injektionsvorrichtung zusammenzuwirken, die während eines Injektionsprozesses einen Klickton erzeugt, wobei die Einheit konfiguriert ist zum:

- Aufzeichnen und Zeitstempeln, durch das Mikrofon und die Prozesseinheit, während des zu überwachenden Injektionsprozesses, eines Probetons y, der von einem Vorrichtungsereignis stammt,
- Bestimmen eines ersten und eines zweiten linearen Messübereinstimmungswahrscheinlichkeitsverhältnisses LLR$^{(j)}$, das bezeichnend für eine Übereinstimmung des Probetons mit einem ersten und einem zweiten Referenzton y$^{(j)}$ ist, die jeweils kennzeichnend für ein erstes und ein zweites Injektionsereignis $j$ sind, die während einer Referenzausführung des Injektionsprozesses nacheinander auftreten, aus einem jeweiligen Satz von Modellparametern, die in der elektronischen Einheit gespeichert und aus dem entsprechenden Referenzton identifiziert sind;
- Identifizieren des Vorrichtungsereignisses als ein erstes Ereignis, falls die erste Messübereinstimmung die zweite Messübereinstimmung überschreitet, und umgekehrt;
- Speichern des Zeitstempels zusammen mit einem Bezeichner des identifizierten Ereignisses.

**11.** Elektronische Einheit nach Anspruch 10, **dadurch gekennzeichnet, dass** die Einheit in eine mobile Vorrichtung eingebettet ist und dass die Mikrofon- und Prozesseinheit mit der mobilen Vorrichtung gemeinsam genutzt werden, wobei die mobile Vorrichtung in der Nähe der Injektionsvorrichtung platziert werden kann, zum Überwachen des Injektionsvorgangs.

**12.** Elektronische Einheit nach Anspruch 10, **dadurch gekennzeichnet, dass** die Einheit in die Injektionsvorrichtung oder in ein elektronisches Modul mit einem Modulgehäuse integriert ist, das angepasst ist, um an der Injektionsvorrichtung entfernbar angebracht zu werden, und dass es zwei Mikrofone umfasst.

**13.** Elektronische Einheit nach Anspruch 10, **dadurch gekennzeichnet, dass** die Einheit in die Injektionsvorrichtung oder in ein elektronisches Modul mit einem Modulgehäuse integriert ist, das angepasst ist, um an der Injektionsvorrichtung entfernbar angebracht zu werden, und dass es einen Statusanzeiger (20c) zum Anzeigen eines Status des Systems an einen Benutzer, insbesondere zum Anzeigen eines Ablaufs einer Haltezeit, umfasst.

**14.** Elektronische Einheit nach Anspruch 10, **dadurch gekennzeichnet, dass** die Einheit in die Injektionsvorrichtung oder in ein elektronisches Modul mit einem Modulgehäuse integriert ist, das angepasst ist, um an der Injektionsvorrichtung entfernbar angebracht zu werden, **dadurch gekennzeichnet, dass** es eine Datenspeicherungseinheit (44) zum Speichern mindestens einer Zeitaufnahme und von Qualitätsinformationen eines Injektionsereignisses, und eine drahtlose Kommunikationseinheit (43) zum Übertragen der gespeicherten Informationen an eine mobile Vorrichtung (3) umfasst.

**15.** Computerprogramm, das, wenn es durch eine Prozesseinheit einer mobilen Vorrichtung zum Überwachen eines mittels einer Injektionsvorrichtung durchgeführten Injektionsprozesses ausgeführt wird, bewirkt, dass die Prozesseinheit nach einem der Ansprüche 1 bis 8 arbeitet.


**Revendications**

**1.** Procédé de surveillance d'un processus d'injection exécuté au moyen d'un dispositif d'injection (1), comprenant

- l'enregistrement d'un premier et d'un deuxième son de référence y$^{(j)}$caractéristique, respectivement, d'un premier et d'un second événement d'injection $j$ se produisant successivement pendant l'exécution de référence du processus d'injection ;
- l'enregistrement, par un microphone (41) et une unité de traitement (42) connectée au microphone, pendant le processus d'injection à surveiller, d'un son échantillon $y$ provenant d'un événement de dispositif, et la génération d'un horodatage indiquant un moment d'enregistrement du son d'échantillon ;
- la détermination d'un premier et d'un second rapport de vraisemblance linéaire, LLR$^{(j)}$, de mesure de correspondance indiquant une correspondance du son d'échantillon avec le premier et le deuxième son de référence, respectivement ;
- l'identification de l'événement de dispositif en tant que premier événement d'injection si la première mesure de correspondance dépasse la deuxième mesure de correspondance, et l'identification de l'événement de dispositif en tant que second événement d'injection si la deuxième mesure de correspondance dépasse la première mesure de correspondance ;
- le stockage de l'horodatage conjointement avec un identifiant $j^*$ de l'événement d'injection identifié.

**2.** Procédé selon la revendication 1, comprenant,

- l'identification, à partir des premier et deuxième sons de référence, d'un premier et d'un second ensemble $\theta^{(j)}$ de paramètres de modèle respectifs, et
- la détermination des première et deuxième mesures de correspondance sur la base du premier et du second ensemble de paramètres de modèle, respectivement.

**3.** Procédé selon la revendication 2, dans lequel un nombre $N^{(1)}$ de paramètres dans le premier ensemble de paramètres de modèle est différent d'un nombre $N^{(2)}$ de paramètres dans le second ensemble.

**4.** Procédé selon la revendication 2, comprenant

- la génération, à partir du son d'échantillon, d'un signal à temps discret $y$ avec un nombre $K$ de points de données, chaque point de données comprenant le temps $k$ et les données correspondantes $y_k$ ;
- la détermination, pour chaque point de données du signal à temps discret, d'une mesure de correspondance locale entre les données correspondantes $y_k$ du signal à temps discret et une évaluation du premier ensemble $\theta^{(l)}$ de paramètres de modèle au temps $k$, et
- la détermination de la première mesure de correspondance en tant que mesure de correspondance locale maximale.

**5.** Procédé selon la revendication 2, dans lequel les paramètres de modèle comportent une fréquence $\omega_l$ et un facteur de décroissance $\rho_l$ d'une matrice $A$ de rotation de mise à l'échelle d'un modèle d'espace d'état linéaire du premier et du deuxième son de référence.

**6.** Procédé selon l'une des revendications 1 à 5, dans lequel le premier événement d'injection est un début d'un processus d'expulsion de médicament signalé par un clic de début d'injection ; Sol ; et le second événement d'injection est une fin du processus d'expulsion de médicament signalée par un clic de fin d'injection, Eol, le procédé comprenant en outre

- l'identification de l'événement de dispositif en tant que début d'un processus d'expulsion de médicament,
- l'enregistrement, par la suite, d'un son d'échantillon supplémentaire provenant d'un événement de dispositif supplémentaire, la génération d'un horodatage indiquant un moment d'enregistrement de l'autre son d'échantillon, et l'identification de l'événement de dispositif supplémentaire en tant que fin du processus d'expulsion de médicament,
- l'évaluation d'un retard temporel entre les horodatages de la fin et du début du processus d'expulsion de médicament pour conclure à une exécution du processus d'expulsion de médicament réussie.

**7.** Procédé selon la revendication 6, dans lequel le dispositif d'injection est un auto-injecteur avec un ressort d'injection pour expulser une dose fixe de médicament à partir d'une seringue (13a) incluse dans le dispositif d'injection, et avec un manchon de couvercle (11a) sollicité par un ressort de manchon de couvercle pour protéger une aiguille de la seringue, le procédé comprenant

- l'enregistrement d'une troisième caractéristique sonore de référence du manchon de couvercle étant verrouillé dans une position de protection d'aiguille ;
- l'enregistrement, par le microphone et l'unité de traitement, pendant le processus d'injection à surveiller, d'un son d'échantillon suivant provenant d'un événement de dispositif suivant, et la génération d'un horodatage indiquant un moment d'enregistrement du son d'échantillon suivant ;
- la détermination d'une première, d'une deuxième et d'une troisième mesure de correspondance indiquant une correspondance du son d'échantillon suivant avec les premier, deuxième et troisième sons de référence, respectivement ;
- l'identification de l'événement de dispositif suivant en tant que verrouillage de manchon de couvercle dans une position de protection si la troisième mesure de correspondance dépasse la première et la deuxième mesure de correspondance,
- l'évaluation d'un retard temporel entre l'horodatage de l'événement de verrouillage de manchon de couverture et un horodatage de la fin du processus d'expulsion de médicament pour conclure à un temps de maintien suffisant.

**8.** Procédé selon l'une des revendications 1 à 5, dans lequel le dispositif d'injection est un dispositif d'injection à dose variable générant des clics de réglage et d'expulsion, dans lequel le premier événement d'injection est un événement de réglage d'unité de dosage signalé par un clic de réglage, et le second événement d'injection est un événement d'expulsion d'unité de dosage signalé par un clic d'expulsion, le procédé comprenant

- l'identification de chacun d'une série d'événements de dispositif en tant qu'événement d'expulsion d'unité de dosage,
- le comptage du nombre d'événements d'expulsion d'unité de dosage dans la série pour déterminer une dose expulsée.

**9.** Procédé selon l'une des revendications 6 à 8, comprenant

- la dérivation, à partir d'une pluralité d'horodatages stockés et d'identifiants d'événement d'injection, d'informations d'injection consolidées concernant le processus d'injection comportant une information de qualité du processus d'injection, et
- la transmission, par une unité de communication sans fil (43) connectée à l'unité de traitement, des informations d'injection consolidées à un dispositif mobile (31).

**10.** Unité électronique comprenant un microphone (41) et une unité de traitement (42) connectée au microphone, configurée pour coopérer avec un dispositif d'injection générant un son de clic pendant un processus d'injection, l'unité étant configurée pour

- enregistrer et horodater, par le microphone et l'unité de traitement, pendant le processus d'injection à surveiller, un son d'échantillon $y$ provenant d'un événement de dispositif,
- déterminer un premier et un second rapport de vraisemblance linéaire, $LLR^{(j)}$, de mesure de correspondance indiquant une correspondance du son d'échantillon avec un premier et un deuxième son de référence $y^{(j)}$ caractéristique respectivement d'un premier et d'un second événement d'injection $j$ se produisant successivement pendant une exécution de référence du processus d'injection, à partir d'un ensemble respectif de paramètres de modèle stockés dans l'unité électronique et identifiés à partir du son de référence correspondant ;
- identifier l'événement de dispositif en tant que premier événement si la première mesure de correspondance dépasse la deuxième mesure de correspondance, et vice versa ;
- stocker l'horodatage conjointement avec un identificateur de l'événement identifié.

**11.** Unité électronique selon la revendication 10, **caractérisée en ce que** l'unité est intégrée dans un dispositif mobile, et **en ce que** le microphone et l'unité de traitement sont partagés avec le dispositif mobile, le dispositif mobile pouvant être placé à proximité du dispositif d'injection pour surveiller le processus d'injection.

**12.** Unité électronique selon la revendication 10, **caractérisée en ce que** l'unité est intégrée au dispositif d'injection ou dans un module électronique avec un boîtier de module adapté pour être fixé de manière amovible au dispositif d'injection, et **en ce qu'**elle comporte deux microphones.

**13.** Unité électronique selon la revendication 10, **caractérisée en ce que** l'unité est intégrée dans le dispositif d'injection ou dans un module électronique avec un boîtier de module adapté pour être fixé de manière amovible au dispositif d'injection, et **en ce qu'**elle comprend un indicateur d'état (20c) pour indiquer un état du système à un utilisateur, en particulier pour indiquer un laps de temps de maintien.

**14.** Unité électronique selon la revendication 10, **caractérisée en ce que** l'unité est intégrée dans le dispositif d'injection ou dans un module électronique avec un boîtier de module adapté pour être fixé de manière amovible au dispositif d'injection, **caractérisée en ce qu'**elle comprend une unité de stockage de données (44) pour stocker au moins une information de temps et de qualité d'un événement d'injection, et une unité de communication sans fil (43) pour transmettre les informations stockées à un dispositif mobile (3).

**15.** Programme informatique qui, lorsqu'il est exécuté par une unité de traitement d'un dispositif mobile pour surveiller un processus d'injection réalisé au moyen d'un dispositif d'injection, amène l'unité de traitement à fonctionner selon l'une des revendications 1 à 8.

Fig. 1

Fig. 2

Fig. 3

Fig. 4

Fig. 5

Fig. 6

Fig. 7

Fig. 8

Fig. 9

$k^{*(1)}$

$k^{*(3)}$

$K$

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2781230 A
- WO 2007107564 A
- WO 2013034716 A
- WO 2015006701 A
- WO 2014064691 A
- WO 2016118736 A1
- EP 2812055 A

**Non-patent literature cited in the description**

- **H.-A. LOELIGER et al.** The factor graph approach to model-based signal processing. *Proceedings of the IEEE,* 2007, vol. 95 (6), 1295-1322